(19) Europäisches Patentamt
European Patent Office
Office européen des brevets

(11) Publication number: 0 047 562
B1

(12) EUROPEAN PATENT SPECIFICATION

(45) Date of publication of patent specification: 23.11.83

(21) Application number: 81200987.6

(22) Date of filing: 03.09.81

(51) Int. Cl.³: C 07 C 43/164, C 07 C 41/18, C 07 C 15/107

# (54) Method for the production of alkyl and alkoxyalkyl substituted benzene compounds.

(30) Priority: 05.09.80 US 184506

(43) Date of publication of application: 17.03.82 Bulletin 82/11

(45) Publication of the grant of the patent: 23.11.83 Bulletin 83/47

(84) Designated Contracting States: BE CH DE FR GB IT LI NL

(56) References cited:
FR - A - 1 596 646
GB - A - 1 519 635

ANGEWANDTE CHEMIE, vol. 86, 1974, Weinheim VON ECKEHARD V. DEHMLOW "Phasentransferkatalytische Zwischenphasenreaktionen in der präparativen organischen Chemie" pages 187-196

(73) Proprietor: STAUFFER CHEMICAL COMPANY
Westport Connecticut 06880 (US)

(72) Inventor: Robbins, Jeffrey Dean
2 The Uplands
Berkeley California (US)

(74) Representative: Urbanus, Henricus Maria, Ir. et al,
c/o Vereenigde Octrooibureaux Nieuwe Parklaan 107
NL-2587 BP 's-Gravenhage (NL)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 047 562 B1

Courier Press, Leamington Spa, England.

Method for the production of alkyl and alkoxyalkyl substituted benzene compounds

## Background and Prior Art

This application relates to a new method for production of alkyl and alkoxyalkyl substituted benzene compounds, and more particularly of di-substituted benzene compounds having an alkyl or alkoxyalkyl substituent on one position in the ring, and another substituent at a second position on the ring.

More particularly, this application relates to processes for producing compounds having the formula

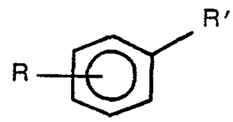

in which R is $C_1$—$C_5$ alkyl, $C_1$—$C_5$ alkoxy, methylenedioxy or hydrogen and R′ is $C_2$—$C_{20}$ alkyl or $C_2$—$C_{20}$ alkoxyalkyl. More preferably, R is $C_1$—$C_5$ alkyl and R′ is $C_{10}$—$C_{15}$ alkoxyalkyl. Most preferably, the compounds have the formula

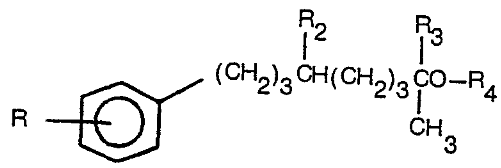

in which R is as defined above, and is located at the para position on the phenyl ring, $R_2$ and $R_3$ are independently methyl or ethyl, and $R_4$ is an alkyl group having from 1 to 4 carbon atoms. Compounds of this type are sometimes referred to as arylterpenoid compounds and have been found useful for regulating and controlling the growth, and particularly the maturation, of insects.

In U.S. Patent 4,002,769, there is described a series of such compounds and a method for their preparation. The method described therein requires five steps, namely:

a) reaction of a substituted benzyl alcohol with hydrogen chloride to produce the corresponding substituted benzyl chloride:

b) reaction of the benzyl chloride with triphenylphosphine in the presence of acetonitrile to produce a benzyl triphenylphosphonium chloride;

c) reaction of the chloride with an olefinically unsaturated aldehyde in the presence of ethanol and sodium ethoxide to produce a phenyl substituted diene;

d) the combination of alkoxy mercuration and demercuration of this diene using mercuric acetate, an alcohol and a mixture of sodium hydroxide and sodium borohydride, to produce an alkoxyolefinically substituted phenyl compound; and finally,

e) hydrogenation of the olefinic bond with, for example, a platinum catalyst, producing an alkoxy substituted phenyl compound (i.e., an arylterpenoid compound).

The process involves a lengthy sequence of steps and expensive reagents such as triphenylphosphine and sodium borohydride, and produces a multitude of by-products, all which must be removed and disposed of.

According to FR—A 1,596,646 there is disclosed a process of preparing benzyl cyanides and homologs comprising reacting a solution or aqueous suspension of an alkali metal cyanide with an arylalkylhalide at 70—150°C.

A similar reaction is disclosed in Angewandte Chemie, vol. 86, 1974, by Eckehard V. Dehmlow "Phasentransfer-katalysierte Zweiphasenreaktionen in der präparativen organischen Chemie", pages 187—196, where the preparation of 1-cyano octane is described by allowing 1-chloro-octan to react with aqueous sodium cyanide in the presence of 1.3 mol% tributyl-hexadecylphosphonium bromide as a phase-transfer catalyst (see page 187, Introduction 1.1, point 3).

Moreover, in said article the alkylation of benzylcyanide in the presence of concentrated sodium hydroxide and a small proportion of triethylbenzylammoniumchloride as a phase-transfer catalyst is referred to (cf. page 187, Introduction 1.1, point 2).

## Summary of the Invention

According to the present invention, there is provided a three-step process for production of substituted benzene compounds, and more particularly disubstituted benzene compounds having the formula

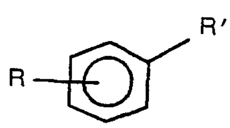

in which R is $C_1$—$C_5$ alkyl, $C_1$—$C_4$ alkoxy, methylenedioxy or hydrogen and R′ is $C_2$—$C_{20}$ alkyl or $C_2$—$C_{20}$ alkoxyalkyl, comprising the steps of

a) reacting a benzyl halide having the formula

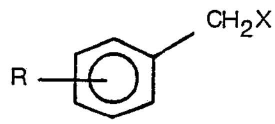

in which X is chlorine, bromine or iodine, with an alkali metal cyanide to produce the corresponding benzyl cyanide;

b) reacting the benzyl cyanide produced in step (a) with a compound having the formula $R_1Y$ in which $R_1$ is $C_1$—$C_{19}$ alkyl or $C_1$—$C_{19}$ alkoxyalkyl and Y is chlorine or bromine, in the presence of a stoichiometric excess of an alkali metal hydroxide, water and a phase transfer catalyst, to produce a compound having the formula

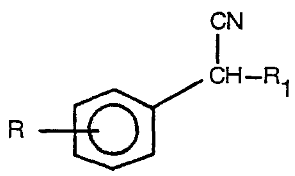 ; and

c) reacting the product of step (b) with an alkali metal and a compound having the formula $R_5OH$ in which $R_5$ is an alkyl group having from 1 to 5 carbon atoms.

More particularly, this invention relates to a process for production of compounds having the formula

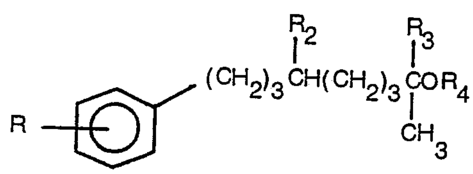

in which R is $C_1$—$C_5$ alkyl, $C_1$—$C_4$ alkoxy, methylenedioxy or hydrogen, $R_2$ and $R_3$ are independently methyl or ethyl and $R_4$ is $C_1$—$C_4$ alkyl, comprising the steps of

a) reacting a compound having the formula

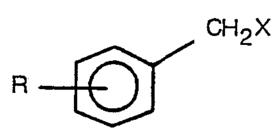

in which X is chlorine, bromine or iodine, with an alkali metal cyanide, producing the corresponding benzyl cyanide;

b) reacting the benzyl cyanide produced in step (a) with a compound having the formula

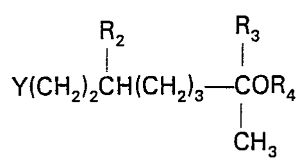

in which Y is chlorine or bromine, in the presence of an alkali metal hydroxide, water and a phase transfer catalyst, to produce a compound having the formula

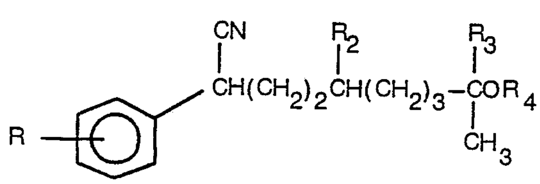 ; and

c) reacting the product of step (b) with an alkali metal and a compound having the formula $R_5OH$ in which $R_5$ is an alkyl group having from 1 to 5 carbon atoms.

Detailed Description of the Invention

As used in this Application, the term "alkyl" includes both straight and branched-chain moieties of this type, in which the total number of carbon atoms is as indicated. The term "alkoxyalkyl" refers to two alkyl groups, which may independently be straight or branched-chain, joined by an oxygen atom, in which the total number of carbon atoms is as indicated.

In the first step of the three-step process to which this invention relates, a benzyl halide having the formula

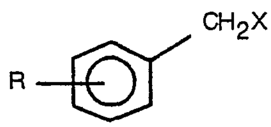

is reacted with an alkali metal cyanide such as sodium or potassium cyanide, and preferably sodium cyanide, to displace the halide moiety by the cyano group. This reaction is preferably conducted in the presence of an organic solvent such as benzene, toluene or xylene, a phase transfer catalyst and sufficient water to dissolve the cyanide. Alternatively, the reaction can be carried out in refluxing aqueous lower alkanol such as methanol, a propanol, or preferably ethanol, in which case a phase transfer catalyst is not necessary. By-product alkali metal chloride is readily removed by washing the reaction product with water.

In the conduct of this first reaction, there may be produced some quantity of the benzyl alcohol corresponding to the benzyl halide used as a starting material, by reaction of the latter with water or OH— ion. The removal of ethanol and such benzyl alcohol, if produced, is highly recommended because the presence of an alcohol is known to inhibit a phase transfer catalyzed alkylation of aryl acetonitriles, as performed in the second step of this process. Removal of the alcohols is performed by appropriate extraction and/or distillation before proceeding to the second step.

Benzyl halides in which R is hydrogen are generally available from commercial sources. Ring-substituted benzyl halides (R is alkyl, alkoxy or methylenedioxy), if not commercially available, can be prepared, for instance, by reaction of the appropriate substituted benzene with formaldehyde and hydrogen chloride, using a zince chloride catalyst.

The second reaction, the alkylation of the benzyl cyanide, is carried out in the presence of excess alkali metal hydroxide, preferably sodium hydroxide, water, and a phase transfer catalyst as described below. The use of the phase transfer catalyst strongly favors monoalkylation of the benzyl cyanide over dialkylation, which is undesirable. In addition, it is not necessary to utilize a solvent for the organic phase, and stronger, expensive, air- or water-sensitive bases are not required.

The preferred alkylating agents of this second step are comparatively long-chain alkoxyalkyl halides of the formula

$$\begin{array}{ccc} & R_2 & & R_3 \\ & | & & | \\ Y(CH_2)_2 & CH & (CH_2)_3— & COR_4 \\ & & & | \\ & & & CH_3 \end{array}$$

in which Y is chlorine or bromine, $R_2$ and $R_3$ are independently methyl or ethyl and $R_4$ is an alkyl group having from 1 to 4 carbon atoms. A preferred member of this group is 7-methoxycitronellyl chloride (Y is chlorine, $R_2$, $R_3$ and $R_4$ are each methyl). This compound may be prepared, for instance, by reaction of methoxycitronellol with phosgene and dimethylformamide. In general, this reaction is conducted at temperatures of from about 0°C to about 100°C., preferably from about 20°C to about 80°C, for a period generally ranging from about 1 to about 10 hours, preferably from about 1 to about 7 hours. The alkali metal hydroxide is preferably used in a 50 percent aqueous solution.

The third step, namely the decyanation of the second stage product, is carried out in the presence of an organic solvent such as benzene, ethylbenzene, xylenes, trimethylbenzenes (e.g. mesitylene) or, preferably toluene, under reflux, using excesses of an alkali metal, preferably sodium, and a lower alkanol. Preferred alkanols are ethanol, isopropanol and 2-methyl-2-butanol. As by-products there are produced, for each mole of the desired product, one mole of alkali metal cyanide and one mole of the corresponding alkoxide. Small amounts of a by-product amine (resulting from reduction of the cyano group instead of reductive cleavage) may be formed. Such by-products can be readily removed by washing the product with aqueous solutions of strong acids.

The phase transfer catalyst used in the first step may be selected from a number of substances which have been found satisfactory for phase transfer catalysis. In general, these compounds fall into four classes: quaternary onium salts, long-chain primary amines, high molecular weight secondary

amines, and crown ethers. In the second step, quaternary onium, preferably ammonium salts, are preferred.

The quaternary onium salts utilized in this invention are disclosed in a number of publications, including U.S. Patent 3,992,432. Such salts generally have the formula

$$(R_5R_6R_7R_8M)^+Z^-$$

in which $R_5$, $R_6$, $R_7$ and $R_8$ are independently alkyl or alkenyl, M is nitrogen, phosphorus or arsenic and Z is a monovalent anion. The alkyl groups of such salts may be straight-chain or branched-chain groups and preferably contain from 1 to 25 carbon atoms each. The alkenyl groups of this class of compounds preferably contain from 2 to 25 carbon atoms each. If one or more of the groups $R_5$—$R_8$ are alkyl, then the total number of carbon atoms contained in all four groups is preferably from 10 to 70, most preferably from 15 to 70. If all four groups $R_5$—$R_8$ are of the alkenyl type, the total number of carbon atoms contained in these groups is preferably from 10 to 40. The alkyl moieties of the group $R_5$—$R_8$ can be a mixture of alkyl groups, derived from mixtures of long-chain fatty alkyl amines or similar compounds. For M, the most preferred is nitrogen; that is, the compounds are quaternary ammonium salts. Z may be any suitable monovalent anion and is preferably a halide, hydroxyl, bisulfate, perchlorate, or nitrate ion. Most preferably, Z is a halide ion, particularly chloride.

Some ammonium salts of this type, in addition to those disclosed in the above-mentioned U.S. patent, are commercially available from Henkel Corporation, Minneapolis, Minnesota, under the registered trademark Aliquat. A preferred member of this class of catalysts is the compounds methyltricaprylyl ammonium chloride, sold as Aliquat® 336. The term "caprylyl" indicates that this component comprises mixtures of alkyl groups having from 8—10, predominantly 8, carbon atoms.

The higher molecular weight amines suitable for use in these processes include both primary and secondary alkyl amines. The primary amines have the formula

$$R_9NH_2$$

in which $R_9$ is a tertiary alkyl group having from about 18 to about 22 carbon atoms, or a mixture of such groups. The secondary amines have the formula

$$R_{10}R_{11}NH$$

in which $R_{10}$ and $R_{11}$ are branched alkyl groups. These secondary amines have a molecular weight generally between about 350 and about 400. The groups $R_{10}$ and $R_{11}$ may be the same or different alkyl and mixtures of various secondary amines of such molecular weight may be similarly utilized.

One example of a suitable type of primary aliphatic amine is the product "Primene JM—T", a mixture of tertiary alkyl primary amines comprised primarily of such compounds having between 18 and 22 carbon atoms available from Rohm and Haas Company, Philadelphia, Pennsylvania. Its molecular weight may vary from about 259 to about 325. Some suitable secondary aliphatic amines are those sold by Rohm and Haas Company under the designations "Amberlite" LA—1 and LA—2. These substances have molecular weights of 351 to 393 and 353 to 395 respectively and contain highly branched alkyl groups attached to the nitrogen atom.

The crown ethers which are useable in this process are described in the literature, for example in *Tetrahedron Letters* No. 18 (1972), pages 1793—1796. They are macrocyclic polyethers and are commonly named by reference to the total number of atoms forming the macrocyclic ring together with the number of oxygen atoms in that ring. Thus, the macrocyclic polyether whose formal chemical name is 1,4,7,10,13,16-hexaoxacyclooctadecane is designated as 18-crown-6.

## Example

By way of example, the following is a description of the production of the compound 2 - methoxy - 2,6 - dimethyl - 9 - p - (isopropylphenyl)nonane (R is p-isopropyl; $R_2$, $R_3$, $R_4$ are all methyl).

### Reaction (a) Cyanation

In a flask were placed 64.5 grams (g) (1.25 mole) 95% sodium cyanide and 56 milliliters (ml) water. The mixture was warmed at 40°C for five minutes to dissolve most of the sodium cyanide. Then, through a dropping funnel, there was added over a period of 27 minutes, with the temperature maintained constant, a solution of 169 g (1.00 mole) p-isopropylbenzyl chloride in 127 g (157 ml) ethanol. The mixture was heated to reflux temperature (about 81°C), then maintained at this temperature for about three hours. The supernatant red liquid was decanted away and the solids were washed with methylene chloride, which was combined with the red liquid. The solids (primarily sodium chloride) were then discarded as waste. The liquid was stripped under vacuum at about 60°C, then taken up in 150 ml methylene chloride and 150 ml carbon tetrachloride respectively and washed with 20 ml water. The aqueous phase was discarded as waste. The organic phase was washed with 100 ml

of 2.5 percent aqueous sodium chloride, then dried over magnesium sulfate, filtered and stripped under vacuum at 50°C to leave 162.0 g of a red liquid, identified as p-isopropylbenzyl cyanide by infrared (ir) and nuclear magnetic resonance (nmr) spectroscopy and mass spectrometry (ms).

This product was distilled under vacuum at 75—79°C. to separate unreacted ethanol and any by-product p-isopropylbenzyl alcohol.

Reaction (b): Alkylation

In a flask was placed 27 ml 50% aqueous sodium hydroxide, 3.0 g mesitylene (internal standard for gas chromatographic analysis), 0.6 g methyltricaprylyl ammonium chloride (Aliquat® 336) and 17.7 g (0.111 mole) p-isopropylbenzyl cyanide (prepared in the previous step). The temperature was established at 22.5°C. There was then added, over a period of 10 minutes, with stirring 20.7 g (0.100 mole) 7-methoxycitronellyl chloride. The mixture was maintained at about 25°C for about three and one-half hours. Then the temperature was raised to 48—52°C, and the mixture maintained at this temperature for eight and one-half hours. The temperature was then raised to 70°C and held for three hours. At the end of this time, the temperature decreased to about 61—64°C and the mixture was maintained at this temperature for two and one-half additional hours.

The reaction mixture was then cooled to −4°C in a carbon dioxide-isopropanol bath and 100 ml water was added with cooling to keep the temperature below 10°C. There was then added, with stirring, 100 ml toluene. Phase separation of the mixture readily occurred. The aqueous phase was extracted with an additional 50 ml toluene and the organic phase washed respectively with 100 ml water, 100 ml 2N hydrochloric acid and a second portion of 100 ml water. The organic liquid, which had a dark brown color, was retained, and vacuum stripped of solvent to leave 34.1 g of an oil, 2 - methoxy - 2,6 - dimethyl - 8 - cyano - 9(p - isopropylphenyl)nonane, identified by ir, nmr and ms.

Reaction (c): Decyanation

In a flask, blanketed with nitrogen, there was placed 17.5 g (0.76 mole) metallic sodium and 75 ml anhydrous toluene. The mixture was heated to reflux and stirred until the sodium was finely divided. Then there was added dropwise, at a rate sufficient to maintain the mix under vigorous reflux, with stirring, 18.2 g (0.050 mol) of the product of reaction (b) and 22.8 g (29 ml) of absolute isopropanol. When this addition was complete, a second portion of 29 ml of isopropanol was added at the same rate. The mixture was then cooled and 100 ml of 95% ethanol was then added until all the sodium had disappeared.

The reaction mixture was a pale, yellow liquid with a mass of white crystals. The liquid was decanted. The crystals were rinsed twice with toluene and the toluene solutions combined with the organic liquid. This liquid was then stripped of solvent under vacuum at 50°C, then taken up in 200 ml toluene and washed with 100 ml water. The aqueous phase was further washed with 50 ml toluene. The combined organic phase was again washed with 50 ml of water, then with two portions, each 50 ml 4N hydrochloric acid, and again water, followed by washing with 50 ml saturated sodium bicarbonate and again water. The organic phase was then stripped under vacuum at 60°C leaving 15.6 g of a clear, yellow-brown mobile oil, quantitatively analyzed by gas chromatography as containing 83.3 weight percent of the desired product. This corresponds to a yield of 81% based on methoxycitronellyl chloride. The structure of the product was confirmed by ir, nmr, and ms.

Example II

This example illustrates the conduct of reaction (a), namely the cyanation step, using a phase transfer catalyst.

In a flask was placed 42.2 g (0.052 mole) p-isopropylbenzyl chloride, 4.2 g mesitylene and 0.7 g (0.0013 mol) methyltricaprylyl ammonium chloride. The ingredients were well mixed, then 18.4 g (0.375 mole) sodium cyanide was added and the mixture cooled to 0°C.

There was then added 2.5 g water. The temperature rose rapidly and then leveled at 7°C. The mixture was then cooled to 0°C and the temperature maintained at about that value for about two hours. The cooling bath was removed, the temperature was allowed to rise to 25°C and the mixture maintained at that temperature for one and one-half hours. The temperature was then raised to 50°C and the mixture maintained for one hour, then raised to 75°C, and the mixture maintained at that temperature for six additional hours.

The reaction mixture was washed with methylene chloride and water, then dried over magnesium sulfate. Solvent was stripped under vacuum at 40°C leaving 42.4 g of p-isopropylphenylacetonitrile, a red liquid. The structure was confirmed by nmr and ir analysis.

## Claims

1. A process for the production of a compound having the formula

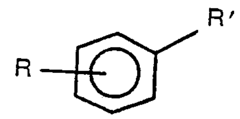

in which R is $C_1$—$C_5$ alkyl, $C_1$—$C_4$ alkoxy, methylenedioxy or hydrogen and R′ is $C_2$—$C_{20}$ alkyl or $C_2$—$C_{20}$ alkoxyalkyl, comprising:

a) reacting a benzyl halide having the formula

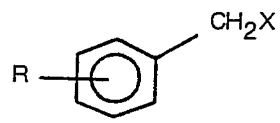

in which X is chlorine, bromine or iodine, with an alkali metal cyanide to produce the corresponding benzyl cyanide;

b) reacting the benzyl cyanide produced in step (a) with a compound having the formula $R_1Y$ in which $R_1$ is $C_1$—$C_{19}$ alkyl or $C_1$—$C_{19}$ alkoxyalkyl and Y is chlorine or bromine in the presence of a stoichiometric excess of an alkali metal hydroxide, water and a phase transfer catalyst to produce a compound having the formula

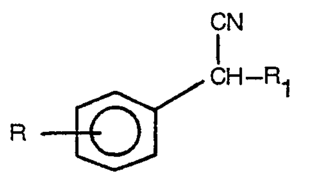

; and

c) reacting the compound produced in step (b) with an alkali metal and a compound having the formula $R_5CH$ in which $R_5$ is an alkyl group having from 1 to 5 carbon atoms.

2. According to Claim 1 in which R′ is

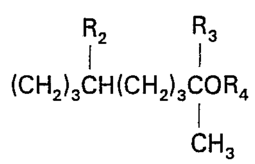

$R_2$ and $R_3$ are independently methyl or ethyl and $R_4$ is $C_1$—$C_4$ alkyl.

3. A process according to Claim 2 in which R is p-isopropyl and $R_2$, $R_3$ and $R_4$ are each methyl.

4. A process according to Claim 1 in which step (a) is conducted in the presence of water, an organic solvent and a phase transfer catalyst.

5. A process according to Claim 4 in which the phase transfer catalyst employed in step (a) is a quaternary onium salt having the formula

$$(R_5R_6R_7R_8M)^+Z^-$$

in which $R_5$, $R_6$, $R_7$ and $R_8$ are independently $C_1$—$C_{25}$ alkyl or $C_2$—$C_{25}$ alkenyl, M is nitrogen, phosphorus or arsenic and Z is a monovalent anion.

6. A process according to Claim 1 in which step (a) is conducted in the presence of an aqueous solution of a lower alkanol.

7. A process according to Claim 1 in which the phase transfer catalyst employed in step (b) is a quaternary onium salt having the formula

$$(R_5R_6R_7R_8M)^+Z^-$$

in which $R_5$, $R_6$, $R_7$ and $R_8$ are independently $C_1$—$C_{25}$ alkyl, or $C_2$—$C_{25}$ alkenyl, M is nitrogen, phosphorus or arsenic and Z is a monovalent anion.

8. A process according to Claim 1 further comprising removing the product of reaction (a) any alcoholic component which may be present before conducting reaction (b).

9. A process according to Claim 1 in which the alkali metal cyanide is sodium cyanide.

10. A process according to Claim 1 in which the alkali metal of step (c) is sodium.

**Revendications**

1. Procédé de production d'un composé répondant à la formule:

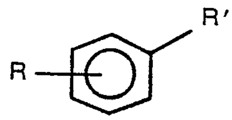

dans laquelle R est un alkyle en $C_1$ à $C_5$, un alkoxy en $C_1$ à $C_4$, un méthylènedioxy ou de l'hydrogène et R′ est un alkyle en $C_2$ à $C_{20}$ ou un alkoxyalkyle en $C_2$ à $C_{20}$, consistant à:

a) faire réagir un halogénure de benzyle répondant à la formule:

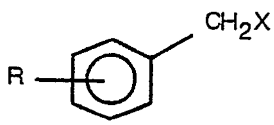

dans laquelle X est du chlore, du brome ou de l'iode avec un cyanure de métal alcalin pour produire le cyanure de benzyle correspondant;

b) faire réagir le cyanure de benzyle obtenu dans l'étape (a) sur un composé répondant à la formule $R_1Y$ dans laquelle $R_1$ est un alkyle en $C_1$ à $C_{19}$ ou un alkoxyalkyle en $C_1$ à $C_{19}$ et Y est du chlore ou du brome en présence d'un excès stoéchiométrique d'un hydroxyde de métal alcalin, de l'eau et un catalyseur de transfert de phase pour obtenir un composé répondant à la formule:

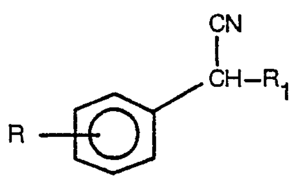

; et

c) faire réagir le composé produit dans l'étape (b) sur un métal alcalin et sur un composé répondant à la formule $R_5OH$ dans laquelle $R_5$ est un groupe alkyle ayant de 1 à 5 atomes de carbone.

2. Procédé selon la revendication 1 dans lequel R′ est

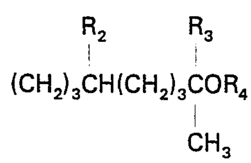

$R_2$ et $R_3$ sont indépendamment du méthyle ou de l'éthyle et $R_4$ est un alkyle en $C_1$ à $C_4$.

3. Procédé selon la revendication 2, caractérisé en ce que R est du p-isopropyle et $R_2$, $R_3$ et $R_4$ sont chacun du méthyle.

4. Procédé selon la revendication 1, caractérisé en ce que l'étape (a) est conduite en présence d'eau, de solvant organique et d'un catalyseur de transfert de phase.

5. Procédé selon la revendication 4, caractérisé en ce que le catalyseur de transfert de phase utilisé dans l'étape (a) est d'un sel d'onium quaternaire répondant à la formule:

$$(R_5R_6R_7R_8M)^+Z^-$$

dans laquelle $R_5$, $R_6$, $R_7$ et $R_8$ sont indépendamment un alkyle en $C_1$ à $C_{25}$ ou un alkényle en $C_2$ à $C_{25}$, M est de l'azote, du phosphore ou de l'arsenic et Z est un anion monovalent.

6. Procédé selon la revendication 1, caractérisé en ce que l'étape (a) est conduite en présence d'une solution aqueuse d'un alcanol inférieur.

7. Procédé selon la revendication 1, caractérisé en ce que le catalyseur de transfert de phase utilisé dans l'étape (b) est un sel d'onium quaternaire répondant à la formule:

$$(R_5R_6R_7R_8M)^+Z^-$$

dans laquelle $R_5$, $R_6$, $R_7$ et $R_8$ sont indépendamment un alkyle en $C_1$ à $C_{25}$, ou un alkényle en $C_2$ à $C_{25}$, M est de l'azote, du phosphore ou de l'arsenic et Z est un anion monovalent.

8. Procédé selon la revendication 1, caractérisé en outre par l'élimination du produit de la réaction (a) de tous les composés alcooliques qui peuvent être présents avant la conduite de la réaction (b).

9. Procédé selon la revendication 1, caractérisé en ce que le cyanure de métal alcalin est du cyanure de sodium.

10. Procédé selon la revendication 1, caractérisé en ce que le métal alcalin de l'étape (c) est le sodium.

**Patentansprüche**

1. Verfahren zur Herstellung einer Alkyl- oder Alkoxyalkyl-substituierten Benzolverbindung der allgemeinen Formel

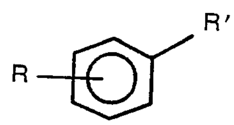

worin R einen $C_1$—$C_5$-Alkyl-, $C_1$—$C_4$-Alkoxy- oder Methylendioxyrest oder ein Wasserstoffatom darstellt und R' einen $C_2$—$C_{20}$-Alkyl- oder $C_2$—$C_{20}$-Alkoxyalkylrest bedeutet, dadurch gekennzeichnet, daß

a) ein Benzylhalogenid der allgemeinen Formel

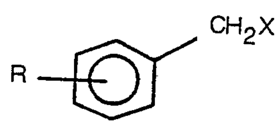

worin X ein Chlor-, Brom- oder Jodatom darstellt, mit einem Alkalimetallcyanid zu dem entsprechenden Benzylcyanid umgesetzt wird,

b) das in Stufe (a) hergestellte Benzylcyanid mit einer Verbindung der allgemeinen Formel

$$R_1Y$$

worin $R_1$ einen $C_1$—$C_{19}$-Alkyl- oder $C_1$—$C_{19}$-Alkoxyalkylrest darstellt und Y ein Chlor- oder Bromatom bedeutet, in Gegenwart eines stöchiometrischen Überschusses eines Alkalimetallhydroxids, Wassers und eines Phasentransferkatalysators zu einer Verbindung der allgemeinen Formel

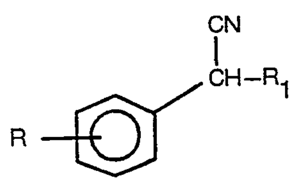

umgesetzt wird, und

c) die in Stufe (b) hergestellte Verbindung mit einem Alkalimetall und einer Verbindung der allgemeinen Formel

$$R_5OH,$$

worin R einen Alkylrest mit 1 bis 5 Kohlenstoffatomen bedeutet, umgesetzt wird.

2. Verfahren nach Anspruch 1, dadurch gekennzeichnet, daß R' den Rest

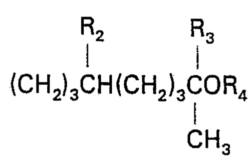

bedeutet, $R_2$ und $R_3$ unabhängig voneinander eine Methyl- oder Ethylgruppe darstellen und $R_4$ einen $C_1$—$C_4$-Alkylrest bedeutet.

3. Verfahren nach Anspruch 2, dadurch gekennzeichnet, daß R für p-Isopropyl steht und $R_2$, $R_3$ und $R_4$ jeweils eine Methylgruppe bedeuten.

4. Verfahren nach Anspruch 1, dadurch gekennzeichnet, daß Stufe (a) in Gegenwart von Wasser, eines organischen Lösungsmittels und eines Phasentransferkatalysators durchgeführt wird.

5. Verfahren nach Anspruch 4, dadurch gekennzeichnet, daß der in Stufe (a) eingesetzte Phasentransferkatalysator ein quaternäres Oniumsalz der allgemeinen Formel

$$(R_5R_6R_7R_8M)^+Z^-$$

ist, worin $R_5$, $R_6$, $R_7$ und $R_8$ unabhängig voneinander jeweils einen $C_1$—$C_{25}$-Alkyl- oder $C_2$—$C_{25}$-Alkenylrest darstellen, M ein Stickstoff-, Phosphor- oder Arsenatom bedeutet und Z ein einwertiges Anion darstellt.

6. Verfahren nach Anspruch 1, dadurch gekennzeichnet, daß Stufe (a) in Gegenwart einer wässrigen Lösung eines niederen Alkanols durchgeführt wird.

7. Verfahren nach Anspruch 1, dadurch gekennzeichnet, daß der in Stufe (b) eingesetzte Phasentransferkatalysator ein quaternäres Oniumsalz der allgemeinen Formel

$$(R_5R_6R_7R_8M)^+Z^-$$

ist, worin $R_5$, $R_6$, $R_7$ und $R_8$ unabhängig voneinander jeweils einen $C_1$—$C_{25}$-Alkyl- oder $C_2$—$C_{25}$-Alkenylrest darstellen, M ein Stickstoff-, Phosphor- oder Arsenatom bedeutet und Z ein einwertiges Anion ist.

8. Verfahren nach Anspruch 1, dadurch gekennzeichnet, daß aus dem Produkt der Umsetzung (a) jegliche alkoholische Komponente, die vorhanden sein kann, vor Durchführung der Reaktion (b) entfernt wird.

9. Verfahren nach Anspruch 1, dadurch gekennzeichnet, daß das Alkalimetallcyanid Natriumcyanid ist.

10. Verfahren nach Anspruch 1, dadurch gekennzeichnet, daß das Alkalimetall der Stufe (c) Natrium ist.